# EUROPEAN PATENT SPECIFICATION

(11) **EP 0 500 758 B1**
(45) Date of publication and mention of the grant of the patent: **23.07.1997**
(21) Application number: 90917681.0
(22) Date of filing: 16.11.1990
(51) Int. Cl.: A61F 13/15

(54) **INCONTINENCE GUARD FOR MEN**
INKONTINENZSCHUTZ FÜR MÄNNER
PROTECTION ANTI-INCONTINENCE POUR HOMMES

(30) Priority: 17.11.1989 SE 8903868
(43) Date of publication of application: 02.09.1992
(73) Proprietor: Mölnlycke AB, 405 03 Göteborg (SE)
(72) Inventor: RUNEMAN, Bo, S-433 75 Partille (SE); RÖNNBERG, Peter, S-431 34 Mölndal (SE)
(74) Representative: Romare, Laila Anette
(86) International application number: SE9000744
(87) International publication number: WO9107155

(56) References cited:
- EP-A- 0 167 931
- WO-A-86/05386
- CH-A- 0 666 612
- SE-B- 0 406 035

## Description

The present invention relates to a male incontinence guard intended for one-time use only and including an inner, liquid-permeable casing layer which is intended to lie closest to the wearer's body in use, an outer, liquid-impermeable casing layer, and an absorbent body or pad located between said casing layers.

Incontinence guards used hitherto have normally consisted of conventional diapers of substantially flat configuration, for instance of rectangular shape. Diapers, however, are intended to absorb both urine and faeces and are therefore not appropriate for use by persons who solely require a urine-collecting incontinence guard. The diaper must have a given width and absorbent material a given thickness in order to achieve satisfactory absorbency. Consequently, the diaper will occupy a correspondingly large space betweed the thighs of the wearer, so as to cause discomfort, such as chafing for instance. Furthermore, there is a serious risk that urine will leak at the edges of the diaper when the diaper becomes saturated and is compressed between the wearer's thighs.

Large, bulky diapers are unacceptable to males who suffer from incontinence but who are otherwise not handicapped. In addition to being uncomfortable when worn, large, bulky diapers are not readily accommodated in conventional clothing.

Mild incontinence is a hidden handicap suffered by many people. A large group of such people is comprised of males with prostate trouble. Subsequent to undergoing prostate surgery, these men are normally troubled by drop-incontinence, which hitherto has resulted in psychic suffering, since no suitable guard is available.

Incontinence guards intended for men afflicted with mild incontinence are known per se. When worn, one such known guard has the form of a cone-shaped container which embraces the genital organs of the male wearer. One drawback with this known guard is that it is much too warm and fits much too tightly when worn, and is therewith uncomfortable to the wearer. A further drawback is that the guard is much too inflexible for comfort.

The Swedish Patent Specification No. SE 450 811 teaches a male incontinence guard which comprises an upper shield-like part which lies over the penis and scrotum of the wearer in use, and a lower part which, in use, curves inwardly beneath the penis and scrotum of the wearer without completely enclosing the same. The guard has a downwardly narrowing and basin-like configuration. This guard thus avoids tightly enclosing the organs of the wearer, which is naturally beneficial from the aspect of comfort.

The guard is formed from a flat blank, one end part of which comprises two flaps whose mutually adjacent edge lines depart from a common point on the guard.

The basin-like configuration is obtained by bringing the flaps to a position in which the edge lines overlap one another, and then joining the flaps in this region.

One drawback with an incontinence guard of this design resides in the complicated manufacturing procedures required by the different clipping and joining steps.

EP-A-0 167 931 describes a disposable absorbent garment for use as a sanitary napkin or incontinence guard. While the possibility of use as a male incontinence guard is mentioned, the construction is designed for women and no consideration has been given to adapt the shape of the garment to suit the particular anatomy of male incontinent persons. The garment comprises a rectangular absorption pad having elastic elements along its longitudinal edges so that the pad can assume a curved shape. This shape will not comfortably embrace the scrotum and penis of a male user, yet stay safely in place and provide satisfactory leakage protection.

WO 86/05386 shows a re-usable incontinence garment having an absorbent pad sewn into a pair of pants. The pad has no elastication. In one embodiment said to be advantageous for men, the pad can have the shape of a truncated triangle. In no other way has the anatomy of male users been considered and in particular, the absorbent pad is not curved to conform to the shape of a male user.

The present invention provides a disposable incontinence guard for males who suffer mild drop-incontinence, with which the drawbacks of the aforesaid incontinence guards are not found.

This is achieved with an incontinence guard in accordance with claim 1.

This results in a compliable incontinence guard which can be worn comfortably. Due to the effect of the elastic devices, the guard will conform more readily to the wearer's genitals than the known guards. Absorbent sanitary products, such as male incontinence guards, must not fit too tightly, feel uncomfortable and be too confining, these requirements being far from unessential. For psychological reasons, the guard should not even be thought to be tightly confining and unpleasant by the presumptive user.

As a result of its construction, an inventive incontinence guard is both simple to manufacture and easy to handle by the user. Triangular absorption pads can be manufactured rationally from a continuous web of absorbent material, without material waste and complicated folding steps.

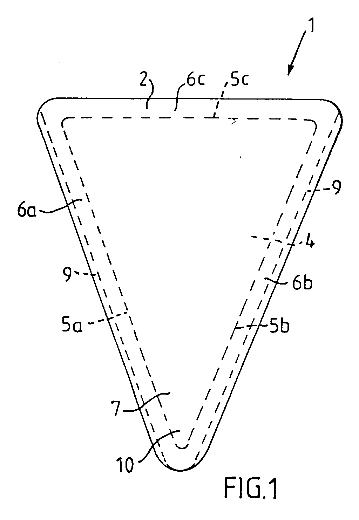

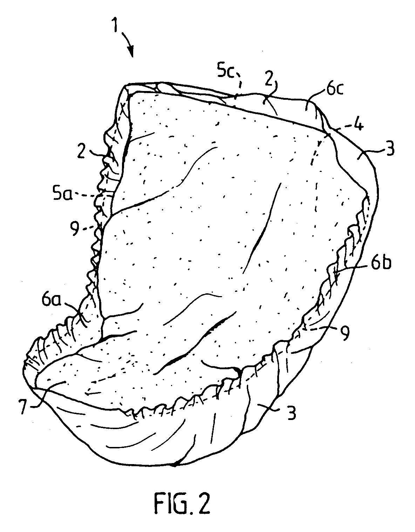

The invention will now be described in more detail with reference to an exemplifying embodiment thereof illustrated in the accompanying drawings, in which Figure 1 illustrates one embodiment of an inventive guard and shows the guard extended with the side intended to lie against the wearer in use facing the viewer, and Figure 2 is a perspective view of the guard illustrated in Figure 1, and shows the elastication in its active state.

The incontinence guard 1 illustrated in Figures 1-2 comprises a liquid permeable layer 2, a liquid impermeable layer 3, and an absorbent pad 4 enclosed between the two layers 2 and 3. The pad 4 may comprise an absorbent fibre material, for instance fluff. If desired, the fluff can be admixed with other absorbent material, for instance so-called superabsorbent polymers, by which is meant polymers that are capable of absorbing liquid in quantities corresponding to many times the natural weight of the polymers. Other substances of a non-absorbent nature can be admixed in the absorbent pad, for instance melt fibres.

The absorbent pad may comprise either one absorbent layer or several absorbent layers, where the various layers may also comprise mutually different absorbent material. The absorbent pad 4 may have the shape of an isosceles triangle, with two edges 5a and 5b of mutually equal length and a third, shorter edge 5c.

The liquid permeable layer 2 is suitably made of non-woven material. Another conceivable material is perforated plastic. The liquid-impermable layer 3 may comprise a polyethylene or polypropylene plastic, or some other liquid impervious plastic. Another conceivable material is hydrophobized non-woven material. The outer layers 2 and 3 will preferably have the same thickness and shape, and both will project slightly beyond the edges 5a, 5b, 5c of the absorbent pad, such as to form side flaps 6a, 6b, 6c along which the layers 2 and 3 are mutually joined with the aid of a binder, for instance melt glue. The equally long edges 5a, 5b of the pad are therewith corresponded by side flaps 6a, 6b of mutually equal length, while the third edge 5c of the absorbent pad is corresponded by a third side flap 6c. The liquid impervious material in the casing 3 is preferably permeable to air and vapours.

Prestretched elastic devices 9 are attached in the side flaps 6a, 6b of mutually equal lengths. The elastic devices 9 may, for instance, have the form of elastic threads, bands or the like. The use of elastic foam material is also conceivable. The elastic devices are preferably glued to one or to both of the outer layers 2, 3. The elastic devices may be placed at any desired distance from the edges 5a, 5b, and the sid flaps 6a, 6b, 6c can have any desired size.

Figure 1 illustrates the incontinence guard in an extended or outwardly stretched form, i.e. with the elastic devices in a prestretched state, whereas Figure 2 illustrates the guard in the form in which it is used, i.e. with the elastic devices in a contracted state. Shortening of the elastic devices by contraction is permitted by curving of the absorbent pad 4. The local resistance of the pad to bending about axes parallel with the edge 5c decreases towards the end 10, and consequently the pad 4 will bend or curve more pronouncedly in the region nearest the end 10 than in parts more distal therefrom. As will be seen from Figure 2, the elastic devices 9 generate a curved, container-like part 7 which is intended to embrace the scrotum of the wearer in use, either completely or partially. Due to bending or curvature of the absorbent pad, pleating of the casing materials caused by the elastic devices will cause the side flaps 6a, 6b to be upstanding in relation to the absorbent pad.

The elastic devices may be prestressed to the same or different degree within different parts of the side flaps 6a, 6b. Naturally, different degrees of elastic pretensioning can be considered, depending on the curvature desired and also on the desired size of the incontinence guard.

According to one preferred embodiment of the invention, the elastic devices are attached in the side flaps 6a, 6b in spaced relationship with the edges 5a, 5b of the absorbent pad. As a result of this arrangement, the elastic devices 9 lift-up the side flaps 6a, 6b so as to form leakage-preventing barriers. Furthermore, the side flaps can be utilized in a simple and practical fashion when putting on the guard. When putting on the guard, the guard is first twisted so as to curve in the opposite direction to that intended when the guard is worn, whereafter the guard is gripped with the fingers of one hand in the region of the pointed end 10, in one region of the side flaps 6a, 6b between the elastic devices 9 and the edges 5a, 5b of the absorbent pad, whereafter the guard is then twisted into position over the wearer's genitals, with the aid of the elastication.

The invention shall not be considered to be limited to the aforedescribed exemplifying embodiments, since several modifications can be made within the scope of the following Claims. For instance, the elastic devices 9 can be mutually joined at the end 10 and may consist of one single elastic thread.

It will also be understood that triangles having rounded corners and triangles having non-linear edges are also conceivable. In order to enable the article to be gripped more readily when putting on the article, the absorbent pad may advantageously be provided with rounded recesses. This arrangement also results in higher sealing barriers, since the distance between the edge of the side flap and the edge of the absorbent pad increases when such recesses are provided.

It will also be understood that some form of absorbent material can also be included in the side flaps. In order to achieve a good elastic seal and enable the article to be twisted readily when putting on the article, the material used in the side flaps must be more flexible and bendable than the material used in the absorbent pad.

In one variant of the inventive article, it is conceivable to provide said article with cross-elastication, so that the absorbent pad will also bend around longitudinally extending axes.

Also conceivable is the use of casing material which will contract and is elastic subsequent to being heated, and to heat this elastication locally instead of using elastic threads and the like which must be held constantly stretched during manufacture of the guard.

## Claims

1. A male incontinence guard intended for one-time use only and including an inner, liquid permeable casing layer (2), which is intended to lie closest to the body of the wearer in use; an outer liquid impermeable casing layer (3); an absorbent pad (4) enclosed between said layers, said pad (4) having a first end (10), an opposing second end and side edges (5a,5b) extending between said first and second ends, and having a longitudinal direction extending between said first (10) and second ends; and at least one elastic device (9) attached in a prestretched state to the inner casing layer (2) and extending along each of the side edges (5a,5b) of said pad over at least a part of the lengths of said side edges so that contraction of said elastic device (9) from said prestretched state causes that part of the absorbent pad located between said side edge parts to take a shape curved in said longitudinal direction; **characterized** in that at least a first part of the absorbent pad (4) narrows towards said first end (10) thereof, intended to be directed towards the back of the wearer, so that within this first part the local resistance of the pad (4) to bending decreases towards said first end (10); and that said at least one elastic device (9) extends from said first, narrower end (10), whereby contraction of the elastic device (9) causes said first part of the absorbent pad (4) to assume a shape which in said longitudinal direction curves more pronouncedly towards said first end (10) and which also curves in a transverse direction perpendicular to said longitudinal direction so that, when the guard is used, said first part will be curved inwardly beneath and surround, at least partially, the scrotum of the wearer.

2. A guard according to claim 1, **characterized** in that the casing layers (2,3) extend somewhat beyond (6a,6b) the side edges (5a,5b) of the absorbent pad; and in that the at least one elastic device (9) is attached between the parts (6a,6b) of the casing layers which extend beyond said side edges.

3. A guard according to claim 1, **characterized** in that the guard and the absorbent pad (4) in the stretched state of the guard have a generally triangular shape.

## Patentansprüche

1. Inkontinenzschutz für Männer, der nur zur einmaligen Verwendung bestimmt ist, und beinhaltend eine innere, flüssigkeitsdurchlässige Hüllenschicht (2), die beim Gebrauch dazu bestimmt ist, eng am Körper des Trägers anzuliegen, eine äußere flüssigkeitsundurchlässige Hüllenschicht (3), ein zwischen den Schichten eingeschlossenes absorbierendes Kissen (4), wobei das Kissen (4) ein erstes Ende (10), ein gegenüberliegendes zweites Ende und Seitenränder (5a, 5b) aufweist, die sich zwischen dem ersten und dem zweiten Ende erstrecken, und das eine sich zwischen dem ersten (10) und dem zweiten Ende erstreckende Längsrichtung besitzt, und zumindest eine elastische Einrichtung (9), die in einem vorgedehnten Zustand an der inneren Hüllenschicht (2) angebracht ist und sich entlang jedes Seitenrands (5a, 5b) des Kissens über zumindest einen Teil der Länge der Seitenränder erstreckt, so daß ein Zusammenziehen der elastischen Einrichtung (9) vom vorgedehnten Zustand aus bewirkt, daß der Teil des absorbierenden Kissens, der zwischen den Seitenrandteilen des absorbierenden Kissens liegt, eine sich in der Längsrichtung krümmende Form einnimmt, **dadurch gekennzeichnet, daß** sich zumindest ein erster Teil des absorbierenden Kissens (4) zu dessen ersten Ende (10) hin, das zur Rückseite des Trägers gerichtet bestimmt ist, verschmälert, so daß innerhalb dieses ersten Teils die lokale Biegefestigkeit des Kissens (4) zum ersten Ende (10) hin abnimmt; und daß sich die zumindest eine elastische Einrichtung (9) von dem ersten, schmäleren Ende (10) aus erstreckt, wodurch ein Zusammmenziehen der elastischen Einrichtung (9) bewirkt, daß das erste Teil des absorbierenden Kissens (4) eine Form einnimmt, die sich in der Längsrichtung zum ersten Ende (10) hin ausgeprägter krümmt und die sich auch in einer senkrecht zur Längsrichtung liegenden Querrichtung krümmt, so daß, wenn der Schutz benutzt wird, sich der erste Teil einwärts, unterhalb und zumindest teilweise um das Skrotum des Trägers krümmen wird.

2. Inkontinenzschutz nach Anspruch 1, **dadurch gekennzeichnet, daß** sich die Hüllenschichten (2, 3) etwas über (6a, 6b) die Seitenränder (5a, 5b) des absorbierenden Kissens erstrecken, und daß die zumindest eine elastische Einrichtung (9) zwischen den Teilen (6a, 6b) der Hüllenschichten angebracht ist, die sich über die Seitenränder erstrecken.

3. Inkontinenzschutz nach Anspruch 1, **dadurch gekennzeichnet, daß** der Schutz und das absorbierende Kissen (4) im gedehnten Zustand des Schutzes eine im wesentlichen dreieckige Form haben.

## Revendications

1. Protection anti-incontinence pour homme, à usage unique et comprenant : une couche d'enveloppe intérieure (2), perméable aux liquides, qui est destinée à se placer, en service, le plus pris du corps de l'utilisateur; une couche d'enveloppe extérieure (3), imperméable aux liquides; un coussin absorbant (4) enfermé entre lesdites couches, ledit coussin (4) comprenant une première extrémité (10), une deuxième extrémité opposée et des bords latéraux (5a, 5b) s'étendant entre lesdites première et deuxième extrémités, et ayant une direction longitudinale qui s'étend entre lesdites première (10) et deuxième extrémités; et au moins un dispositif élastique (9) fixé dans un état de pré-étirement à la couche d'enveloppe intérieure (2) et s'étendant le long de chacun des bords latéraux (5a, 5b) dudit coussin sur une partie au moins de la longueur desdits bords latéraux de sorte qu'une contraction dudit dispositif élastique (9) par rapport audit état de pré-étirement fait que la partie du coussin absorbant située entre lesdites parties des bords latéraux prend une forme courbe dans ladite direction longitudinale, caractérisée en ce qu'une première partie au moins du coussin absorbant (4) se rétrécit en direction de ladite première extrémité (10) de ce dernier, destinée à être dirigée vers le dos de l'utilisateur, de sorte que dans cette première partie la résistance locale du coussin (4) à la flexion diminue en direction de ladite première extrémité (10); et en ce que ledit dispositif élastique (9) au nombre d'au moins un s'étend depuis ladite première extrémité plus étroite (10) si bien qu'une contraction dudit dispositif élastique (9) fait prendre à ladite première partie du coussin absorbant (4) une forme qui est courbée dans ladite direction longitudinale de façon plus prononcée vers ladite première extrémité (10) et qui est également courbée dans une direction transversale perpendiculaire à ladite direction longitudinale de sorte que, lorsque ladite protection est utilisée, ladite première partie va se courber vers l'intérieur sous le scrotum de l'utilisateur et l'entourer, au moins partiellement.

2. Protection selon la revendication 1, caractérisée en ce que les couches d'enveloppe (2, 3) s'étendent quelque peu au-delà (6a, 6b) des bords latéraux (5a, 5b) du coussin absorbant, et en ce que ledit dispositif élastique (9) au nombre d'au moins un est fixé entre les parties (6a, 6b) des couches d'enveloppe qui dépassent desdits bords latéraux.

3. Protection selon la revendication 1, caractérisée en ce que la protection et le coussin absorbant (4) dans l'état de pré-étirement de la protection ont une forme globalement triangulaire.
